# FASCICULE DE BREVET EUROPEEN

(11) **EP 0 692 244 B1**
(45) Date de publication et mention de la délivrance du brevet: **02.04.1997**
(21) Numéro de dépôt: 95401358.7
(22) Date de dépôt: 12.06.1995
(51) Int. Cl.: A61K 7/06, A61K 7/48

(54) **Compositions cosmétiques détergentes et utilisation**
Reinigende kosmetische Zusammensetzungen und ihre Verwendung
Cosmetic detergent compositions and their use

(30) Priorité: 11.07.1994 FR 9408570
(43) Date de publication de la demande: 17.01.1996
(73) Titulaire: L'OREAL, 75008 Paris (FR)
(72) Inventeur: Decoster, Sandrine, F-93800 Epinay sur Seine (FR); Beauquey, Bernard, F-92110 Clichy (FR); Cotteret, Jean, F-78480 Verneuil sur Seine (FR)
(74) Mandataire: Tezier Herman, Béatrice

(56) Documents cités:
- EP-A- 0 469 602
- EP-A- 0 545 786
- EP-A- 0 595 683
- EP-A- 0 600 445
- WO-A-91/14419
- WO-A-93/11103
- WO-A-94/09754
- FR-A- 2 664 162
- GB-A- 2 011 786
- US-A- 4 800 076
- SEIFEN-ÖLE-FETTE-WACHSE, vol. 94, no. 26, pages 931-932, 'kolloidale kieselsäure als mattierungsmittel in flüssigem make-up'

## Description

La présente invention concerne de nouvelles compositions cosmétiques destinées au nettoyage des cheveux, du cuir chevelu et/ou de la peau, à base de tensioactifs détergents, d'agents de conditionnement particuliers et d'argile , ainsi que leur utilisation dans cette application cosmétique.

Pour le nettoyage des cheveux et/ou de la peau, l'utilisation de compositions cosmétiques détergentes (shampooings ou gels-douche par exemple) contenant à la fois des agents tensioactifs à pouvoir lavant et un ou plusieurs agents de conditionnement est courante.

En effet, pour améliorer les propriétés cosmétiques des compositions détergentes, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est souvent nécessaire d'introduire dans ces dernières des agents cosmétiques complémentaires comme par exemple des agents de conditionnement insolubles, qui apportent alors aux cheveux traités une facilité de démêlage et de coiffage, ainsi qu'une douceur et une brillance, nettement accrues.

Compte tenu du caractère insoluble de ces agents de conditionnement dans les milieux aqueux utilisés dans les shampooings, on cherche à maintenir les agents de conditionnement sous forme dispersée. Cette forme dispersée permet à l'agent de conditionnement de se déposer sur les cheveux ou sur la peau et de ne pas être totalement éliminée lors du rinçage. Il importe toutefois que la mise en suspension ne perturbe pas les propriétés détergentes et moussantes de la composition cosmétique.

Il existe à ce jour peu de moyens pour maintenir efficacement en suspension les agents de conditionnement insolubles, car il s'agit là d'un problème difficile à résoudre ; à cet effet, on a déjà proposé l'utilisation des dérivés d'esters ou d'éthers à longue chaîne (agents nacrants) ou des polysaccharides tels que la gomme de xanthane (gélifiants). Cependant, les agents nacrants présentent des problèmes de cristallisation qui entrainent une évolution (augmentation) de la viscosité des compositions au cours du temps ; les agents gélifiant présentent également des inconvénients, à savoir d'une part que la mousse des compositions détergentes contenant de la gomme de xanthane se développe difficilement (mauvais démarrage de mousse) et que, d'autre part, les compositions n'ont pas une texture lisse et s'écoulent par paquets, ce qui est peu apprécié des utilisateurs.

La présente invention a pour but de proposer un nouveau moyen permettant de maintenir en suspension certains agents de conditionnement insolubles dans des shampooings.

Ainsi, à la suite d'importantes recherches menées sur la question, la demanderesse a maintenant découvert qu'en introduisant une argile dans les compositions cosmétiques détergentes à base de tensioactifs et de certains agents de conditionnement insolubles, il est possible de maintenir en suspension lesdits agents de conditionnement insolubles tout en maintenant un pouvoir moussant suffisant.

Les compositions selon l'invention sont stables : en particulier, il ne se produit aucun relargage de l'agent de conditionnement ou d'épaississement incontrôlé de la composition au cours du temps. En outre, les propriétés moussantes, telles que le démarrage de la mousse, ne sont pas diminuées. Les propriétés lavantes et les autres propriétés cosmétiques avantageuses (douceur, démêlage, coiffage) qui sont affachés à ces compositions sont par ailleurs conservées. Les compositions présentent enfin une texture non filante et fondante.

Toutes ces découvertes sont à la base de la présente invention.

Ainsi, selon la présente invention, il est maintenant proposé de nouvelles compositions cosmétiques détergentes, du type comprenant, dans un milieu aqueux cosmétiquement acceptable, au moins un agent tensioactif détergent et au moins un agents de conditionnement insoluble choisi parmi les poly-α-oléfines, les huiles fluorées, les cires fluorées, les gommes fluorées et les esters d'acides gras, et qui sont caractérisées par le fait qu'elles comprennent en outre au moins une argile, le pouvoir moussant étant supérieur à 50 ml.

L'invention a également pour objet l'utilisation en cosmétique des compositions ci-dessus pour le nettoyage des cheveux, de la peau et/ou du cuir chevelu.

Elle a également pour objet l'utilisation d'une argile comme agent de mise en dispersion d'un agent de conditionnement insoluble choisi parmi les poly-α-oléfines, les huiles fluorées, les cires fluorées, les gommes fluorées et les esters d'acides gras dans une composition cosmétique détergente contenant un tel agent de conditionnement et au moins un agent tensioactif détergent.

D'autres caractéristiques, aspects et avantages de l'invention apparaîtront encore plus clairement à la lecture de la description qui va suivre, ainsi que des exemples concrets, mais nullement limitatifs, destinés à l'illustrer.

Le pouvoir moussant de la composition est mesurée selon la méthode Ross Miles (NF T 73-404) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

La nature des tensioactifs rentrant dans les compositions détergentes selon l'invention n'est pas critique, ils peuvent être de type anionique, non ionique, amphotère, zwittérionique ou cationique. De préférence, les agents tensioactifs détergents utilisables selon l'invention sont de type anionique, non ionique, amphotère ou zwittérionique. On utilise de préférence un ou plusieurs tensioactifs anioniques ou encore plus particulièrement un mélange de tensioactifs anioniques et de tensioactifs amphotères, zwittérioniques ou non ioniques.

Ainsi, à titre d'exemple de tensioactifs anioniques utilisables, seuls ou mélanges, dans le cadre de la présente invention, on peut citer notamment les sels (en particulier sels alcalins, notamment de sodium, sels d'ammonium, sels d'amines, sels d'aminoalcools ou sels de magnésium) des composés suivants : les alkylsulfates, les alkyléthersulfates, les alkylamidoéthersulfates, les monoglycérides sulfates les alkylglycérylsulfonates; les alkylsulfonates, les alkylphosphates, les alkylamidesulfonates, les alkylarylsulfonates, les α-oléfinesulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acyliséthionates, les N-acylaminoacides tels que les N-acylsarcosinates, les N-acylglutamates et les N-acyltaurates,. Parmi les tensioactifs anioniques encore utilisables, on peut également citer les sels d'acides gras tels que les sels des acides undécylénique, oléique, ricinoléique, palmitique, stéarique, les acides d'huile de coprah ou d'huile de coprah hydrogénée les acylhydroxyacides tels que les acyl-lactylates. On peut également utiliser des tensioactifs faiblement anioniques, comme les acides d'alkyl D galactoside uronique et leurs sels ainsi que les acides éthers carboxyliques polyoxyalkylénés, en particulier ceux comportant de 2 à 24 groupements oxyde d'éthylène, et leurs mélanges. Le radical alkyle ou acyle de tous ces différents composés comporte de préférence de 8 à 22 atomes de carbone.

A titre de tensioactifs non ioniques, on peut citer les alcools, les alphadiols, les alkylphénols ou les acides gras polyéthoxylés, polypropoxylés ou polyglycérolés, ayant une chaîne grasse comportant de 8 à 28 atomes de carbone, le nombre de groupements d'oxyde d'éthylène ou de propylène pouvant aller de 2 à 50 et celui de glycérol notamment de 2 à 30. On peut également citer les copolymères d'oxyde d'éthylène et de propylène, les condensats d'oxyde d'éthylène et de propylène sur des alcools gras; les amines ou les amides gras polyéthoxylés ayant de préférence de 2 à 30 moles d'oxyde d'éthylène, les amides gras polyglycérolés comportant en moyenne 1 à 5 groupements glycérol, les diglycolamides polyglycérolés, les esters d'acides gras du sorbitan éventuellement oxyéthylénés, les esters d'acides gras du sucrose, les esters d'acides gras polyoxyalkylénés, les alkylpolyglycosides éventuellement oxyalkylénés, les esters d'alkylglucosides, les dérivés de N-alkylglucamine et de N-acylméthylglucamines, les oxydes d'amine.

A titre de tensioactifs amphotères ou zwittérioniques, on peut citer les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer les alkylbétaines, les alkyldiméthylbétaines, les alkylsulfobétaines, les alkylamidoalkylbétaines, les alkylamidoalkylsulfobétaines, les dérivés d'imidazoline tels que les dérivés d'amphocarboxyglycinate ou d'amphocarboxypropionate.

Le ou les tensioactifs sont généralement présents dans les compositions conformes à l'invention dans des proportions comprises entre 5 à 50 % en poids, de préférence de 5 à 20 % en poids, par rapport au poids total de la composition.

On entend ici par agent de conditionnement insoluble tout agent de conditionnement, insoluble ou substantiellement insoluble dans l'eau et/ou dans les milieux aqueux renfermant des tensioactifs.

Les agents de conditionnement peuvent se présenter sous forme liquide, semi-solide ou solide tels que par exemple des huiles, des cires ou des gommes.

Selon l'invention, les agents de conditionnement doivent être choisis parmi les poly-α-oléfines, les huiles fluorées, les cires fluorées, les gommes fluorées et les esters d'acides gras .

Les poly-α-oléfines sont en particulier :
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.
   A titre d'exemples de poly-α-oléfines utilisables dans le cadre de la présente invention, on peut plus particulièrement mentionner les produits vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.
   De tels produits sont vendus par exemple sous les dénominations ETHYLFLO par la société ETHYL CORP., et d'ARLAMOL PAO par la société ICI.

Les esters d'acides gras sont par exemple les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le malate de dioctyle, le laurate d'hexyle, le laurate de 2-hexyldécyle et l'isononanate d'isononyle,
Les huiles fluorées, les cires fluorées, les gommes fluorées sont par exemple les perfluoropolyéthers décrits notamment dans la demande de brevet EP-A-486135 et les composés fluorohydrocarbonées décrites notamment dans la demande de brevet WO 93/11103.

Le terme de composés fluorohydrocarbonées désigne des composés dont la structure chimique comporte un squelette carboné dont certains atomes d'hydrogène ont été substitués par des atomes de fluor.

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Il est bien entendu possible de mettre en oeuvre des mélanges d'agents de conditionnement.

Le ou les agents de conditionnement sont présents dans les compositions conformes à l'invention dans des proportions généralement comprises entre 0,001 et 10% en poids, de préférence de 0,01 à 5 % en poids, et encore plus particulièrement de 0,1 à 2% en poids par rapport au poids total de la composition.

Selon une caractéristique essentielle des compositions cosmétiques détergentes selon l'invention, celles-ci contiennent au moins une argile, naturelle ou synthétique, mais de préférence naturelle.

Les compositions détergentes conformes à l'invention peuvent bien entendu contenir une ou plusieurs argiles.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson".

A titre d'exemples de tels produits, on peut citer les argiles de la famille de la kaolinite telles que la kaolinite, la dickite, la nacrite, les argiles de la famille de l'halloysite, de la dombassite, de l'antigorite, de la berthiérine, de la pyrophyllite, des montmorillonites, de la beidellite, des vermiculites, du talc, de la stévensite, des hectorites, des saponites, des chlorites, de la sépiolite.

Les argiles peuvent également être modifiées chimiquement par divers composés tels que les acides acryliques, les polysaccharides (par exemple la carboxyméthylcellulose) ou les cations organiques.

De préférence, on retiendra les argiles qui sont cosmétiquement compatibles et acceptables avec les cheveux, la peau et/ou le cuir chevelu.

Selon un mode particulièrement préféré de réalisation de la présente invention, l'argile mise en oeuvre est choisie parmi la kaolinite, les montmorillonites, les hectorites On utilise encore plus particulièrement les mélanges d'argiles.

Selon l'invention, la ou les argiles sont présentes dans les compositions généralement à raison de 0,5 à 15% en poids, et particulièrement de 2 à 10 % en poids, encore plus préférentiellement de 4 à 8% en poids, par rapport à l'ensemble de la composition.

Le véhicule, ou support, des compositions détergentes selon l'invention est de préférence de l'eau ou une solution hydroalcoolique d'un alcool inférieur tel que éthanol, isopropanol ou butanol.

Les compositions détergentes selon l'invention présentent un pH final généralement compris entre 4 et 8. De préférence, ce pH est compris entre 5 et 7. L'ajustement du pH à la valeur désirée peut se faire classiquement par ajout, selon les cas, soit d'agents basifiants, soit d'agents acidifiants, usuels et connus comme cosmétiquement acceptables.

Les compositions détergentes selon l'invention peuvent bien entendu contenir en outre des adjuvants choisis parmi ceux habituellement rencontrés dans le domaine des shampooings pour les cheveux et/ou pour le corps, comme par exemple des parfums, des agents conservateurs, des séquestrants, des agents acidifiants, des agents alcalinisants, des épaississants autres que l'argile, des adoucissants, des modificateurs de mousse, des colorants, des agents nacrants, des agents hydratants, des agents antipelliculaires ou anti-séborrhéiques, des vitamines, des filtres solaires, des silicones, des tensioactifs de préférence de type cationique, des polymères de préférence cationiques ou amphotères et autres.

Ces compositions peuvent se présenter sous la forme de liquides plus ou moins épaissis, de crèmes ou de gels et elles conviennent principalement au lavage des cheveux et/ou de la peau.

Des exemples concrets illustrant l'invention vont maintenant être donnés.

### EXEMPLE 1

On a préparé un shampooing conforme à l'invention qui présentait la composition suivante :

| | |
|---|---|
| - Lauryl éther sulfate de sodium à 2,2 moles d'oxyde d'éthylène | 9,8 g |
| - Cocoamidoéthyl (N-hydroxyéthyl, N-carboxyméthyl) glycinate de sodium, vendu sous le nom de MIRANOL C2M par RHONE-POULENC | 3,8 g |
| - Polyisobutène hydrogéné (2,2,4,4,6,6,8-heptaméthylnonane) vendu sous la dénomination ARLAMOL HD par la société ICI | 0,2 g |
| - Kaolinite (KAOLIN SUPREME 1 de ETS ERNEST ORTMANS) | 3 g |
| - Montmorillonite (GELWHITE HNF de ECC INTERNATIONAL) | 3 g |
| - Conservateurs qs | |
| - Eau qsp | 100 g |

Le pH de ce shampooing a été ajusté à pH 5 par ajout d'acide citrique.

Cette composition de shampooing est stable, la poly-α-oléfine est parfaitement maintenue en suspension et la viscosité n'évolue pas au cours du temps.
On a appliqué cette composition sur des cheveux mouillés, on a massé les cheveux, la mousse s'est développé immédiatement contrairement à une composition renfermant 1% de gomme de xanthane à la place de l'argile.
Le pouvoir moussant de la composition est supérieur à 100 ml.

### EXEMPLE 2

On a préparé un shampooing conforme à l'invention qui présentait la composition suivante :

| | |
|---|---|
| - Lauryl éther sulfate de sodium à 2,2 moles d'oxyde d'éthylène | 9,8 g |
| - Cocoamidoéthyl (N-hydroxyéthyl, N-carboxyméthyl) glycinate de sodium, vendu sous le nom de MIRANOL C2M par RHONE-POULENC | 3,8 g |
| - Kaolinite (KAOLIN SUPREME 1 de ETS ERNEST ORTMANS) | 8 g |
| - Polydécène hydrogéné vendu sous la dénomination ETHYLFLO 364 NF par la société ETHYL CORP. | 1 g |
| - Copolymère acrylate d'alkyle (C₁₀/C₃₀) / acide acrylique réticulé (CARBOPOL 1382 de GOODRICH) | 0,4 g |
| - Conservateurs, colorants, parfum qs | |
| - Eau qsp | 100 g |

Le pH de ce shampooing a été ajusté à pH 5 par ajout d'acide citrique.

Cette composition de shampooing présente les mêmes propriétés que celle de l'exemple 1.

### EXEMPLE 3

On a préparé un shampooing conforme à l'invention qui présentait la composition suivante :

| | |
|---|---|
| - Lauryl éther sulfate de sodium à 2,2 moles d'oxyde d'éthylène | 9,8 g |
| - Cocoamidoéthyl (N-hydroxyéthyl, N-carboxyméthyl) glycinate de sodium, vendu sous le nom de MIRANOL C2M par RHONE-POULENC | 3,8 g |
| - Polydécène hydrogéné vendu sous la dénomination ETHYLFLO 364 NF par la société ETHYL CORP. | 0,2 g |
| - Kaolinite (KAOLIN SUPREME 1 de ETS ERNEST ORTMANS) | 3 g |
| - Montmorillonite (GELWHITE HNF de ECC INTERNATIONAL) | 3 g |
| - Conservateurs qs | |
| - Eau qsp | 100 g |

Le pH de ce shampooing a été ajusté à pH 5 par ajout d'acide citrique.

Cette composition de shampooing présente les mêmes propriétés que celle de l'exemple 1.

### EXEMPLE 4

On a préparé un shampooing conforme à l'invention qui présentait la composition suivante :

| | |
|---|---|
| - Lauryl éther sulfate de sodium à 2,2 moles d'oxyde d'éthylène | 9,8 g |
| - Cocoamidoéthyl (N-hydroxyéthyl, N-carboxyméthyl) glycinate de sodium, vendu sous le nom de MIRANOL C2M par RHONE-POULENC | 3,8 g |
| - Perfluoropolyméthylisopropyl éther vendu sous la dénomination FOMBLIN HC/04 par la société MONTEDISSON | 2 g |
| - Kaolinite (KAOLIN SUPREME 1 de ETS ERNEST ORTMANS) | 5 g |
| - Montmorillonite (GELWHITE HNF de ECC INTERNATIONAL) | 2 g |
| - Copolymère acrylate d'alkyle (C₁₀/C₃₀) / acide acrylique réticulé (CARBOPOL 1382 de GOODRICH) | 0,2 g |
| - Conservateurs qs | |
| - Eau qsp | 100 g |

Le pH de ce shampooing a été ajusté à pH 5 par ajout d'acide citrique.

Cette composition de shampooing présente les mêmes propriétés que celle de l'exemple 1.

## Revendications

1. Compositions cosmétiques détergentes comprenant, dans un milieu aqueux cosmétiquement acceptable, au moins un agent tensioactif détergent et au moins un agent de conditionnement insoluble choisi parmi les poly-α-oléfines, les huiles fluorées, les cires fluorées, les gommes fluorées et les esters d'acides gras, caractérisées par le fait qu'elles comprennent en outre au moins une argile, le pouvoir moussant de la composition étant supérieur à 50 ml.

2. Compositions selon la revendication 1, caractérisées par le fait que la teneur en argile est comprise entre 0,5 et 15% en poids par rapport au poids total de la composition.

3. Compositions selon la revendication 2, caractérisées par le fait que ladite teneur est comprise entre 2 et 10% en poids par rapport au poids total de la composition.

4. Compositions selon la revendication 3, caractérisées par le fait que ladite teneur est comprise entre 4 et 8% en poids par rapport au poids total de la composition.

5. Compositions selon l'une quelconque des revendications précédentes, caractérisées par le fait que le ou les tensioactifs détergents sont présents à raison de 5 à 50% en poids, de préférence de 5 à 20 % en poids, par rapport au poids total de la composition.

6. Compositions selon l'une quelconque des revendications précédentes, caractérisées par le fait que le ou les agents de conditionnement sont présents à raison de 0,001 à 10 % en poids par rapport au poids total de la composition.

7. Compositions selon la revendication 6, caractérisées par le fait que le ou les agents de conditionnement sont présents à raison de 0,01 à 5 % en poids par rapport au poids total de la composition.

8. Compositions selon l'une quelconque des revendications précédentes, caractérisées par le fait qu'elles présentent un pH compris entre 4 et 8.

9. Compositions selon l'une quelconque des revendications précédentes, caractérisées par le fait que le ou les agents tensioactifs détergents sont de type anionique, non ionique, amphotère, zwittérionique.

10. Compositions selon l'une quelconque des revendications précédentes, caractérisées par le fait que le ou les agents tensioactifs détergents sont de type anionique.

11. Compositions selon l'une quelconque des revendications précédentes, caractérisées par le fait que les agents tensioactifs détergents sont un mélange de tensioactifs anioniques et de tensioactifs amphotères, zwittérioniques ou non ioniques.

12. Compositions selon l'une quelconque des revendications précédentes, caractérisées par le fait que l'argile est choisie parmi les argiles de la famille de la kaolinite, de l'halloysite, de la dombassite, de l'antigorite, de la berthiérine, de la pyrophyllite, des montmorillonites, de la beidellite, des vermiculites, du talc, de la stévensite, des hectorites, des saponites, des chlorites, de la sépiolite.

13. Compositions selon la revendication précédente, caractérisées par le fait que l'argile est choisie parmi la kaolinite, les montmorillonites, les hectorites et leurs mélanges.

14. Compositions selon l'une quelconque des revendications précédentes, caractérisées par le fait que les poly-α-oléfines sont de type polybutène, hydrogéné ou non, ou de type polydécène, hydrogéné ou non.

15. Compositions selon l'une quelconque des revendications précédentes, caractérisées par le fait que les esters d'acides gras sont choisis parmi les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le malate de dioctyle, le laurate d'hexyle, le laurate de 2-hexyldécyle et l'isononanate d'isononyle.

16. Compositions selon l'une quelconque des revendications précédentes, caractérisées par le fait que les huiles fluorées, les cires fluorées, les gommes fluorées sont choisies parmi les perfluoropolyéthers et les composés fluorohydrocarbonées.

17. Utilisation d'une argile comme agent de mise en dispersion d'un agent de conditionnement choisi parmi les poly-α-oléfines, les huiles fluorées, les cires fluorées, les gommes fluorées et les esters d'acides gras, dans une composition cosmétique détergente contenant au moins un agent tensioactif détergent.

18. Utilisation d'une composition telle que définie à l'une quelconque des revendications 1 à 16 pour le nettoyage des cheveux, du cuir chevelu et/ou de la peau.

## Claims

1. Detergent cosmetic compositions comprising, in a cosmetically acceptable aqueous medium, at least one detergent surface-active agent and at least one insoluble conditioner chosen from poly-α-olefins, fluoro oils, fluoro waxes, fluoro gums and fatty acid esters, characterized in that they additionally comprise at least one clay, the foaming power of the composition being greater than 50 ml.

2. Compositions according to Claim 1, characterized in that the content of clay is between 0.5 and 15% by weight relative to the total weight of the composition.

3. Compositions according to Claim 2, characterized in that the said content is between 2 and 10% by weight relative to the total weight of the composition.

4. Compositions according to Claim 3, characterized in that the said content is between 4 and 8% by weight relative to the total weight of the composition.

5. Compositions according to any one of the preceding claims, characterized in that the detergent surfactant or surfactants are present in an amount of from 5 to 50% by weight, preferably from 5 to 20% by weight, relative to the total weight of the composition.

6. Compositions according to any one of the preceding claims, characterized in that the conditioner or conditioners are present in an amount of from 0.001 to 10% by weight relative to the total weight of the composition.

7. Compositions according to Claim 6, characterized in that the conditioner or conditioners are present in an amount of from 0.01 to 5% by weight relative to the total weight of the composition.

8. Compositions according to any one of the preceding claims, characterized in that they have a pH between 4 and 8.

9. Compositions according to any one of the preceding claims, characterized in that the detergent surface-active agent or agents are of anionic, nonionic, amphoteric or zwitterionic type.

10. Compositions according to any one of the preceding claims, characterized in that the detergent surface-active agent or agents are of anionic type.

11. Compositions according to any one of the preceding claims, characterized in that the detergent surface-active agents are a mixture of anionic surfactants and amphoteric, zwitterionic or nonionic surfactants.

12. Compositions according to any one of the preceding claims, characterized in that the clay is chosen from clays of the family of kaolinite, halloysite, dombassite, antigorite, berthierine, pyrophyllite, montmorillonites, beidellite, vermiculites, talc, stevensite, hectorites, saponites, chlorites and sepiolite.

13. Compositions according to the preceding claim, characterized in that the clay is chosen from kaolinite, montmorillonites, hectorites and mixtures thereof.

14. Compositions according to any one of the preceding claims, characterized in that the poly-α-olefins are of polybutene type, which may or may not be hydrogenated, or of polydecene type, which may or may not be hydrogenated.

15. Compositions according to any one of the preceding claims, characterized in that the fatty acid esters are chosen from ethyl palmitate, isopropyl palmitate, 2-ethylhexyl palmitate, 2-octyldecyl palmitate, alkyl myristates such as isopropyl myristate, butyl myristate, cetyl myristate and 2-octyldodecyl myristate, hexyl stearate, butyl stearate, dioctyl malate, hexyl laurate, 2-hexyldecyl laurate and isononyl isononanate.

16. Compositions according to any one of the preceding claims, characterized in that the fluoro oils, the fluoro waxes and the fluoro gums are chosen from perfluoropolyethers and fluorohydrocarbon compounds.

17. Use of a clay as agent for placing in dispersion a conditioner, chosen from poly-α-olefins, fluoro oils, fluoro waxes, fluoro gums and fatty acid esters, in a detergent cosmetic composition containing at least one detergent surface-active agent.

18. Use of a composition as defined in any one of Claims 1 to 16 for cleaning the hair, the scalp and/or the skin.

## Patentansprüche

1. Kosmetische reinigende Zusammensetzungen, die in einem kosmetisch akzeptablen wäßrigen Medium mindestens ein grenzflächenaktives Reinigungsmittel und mindestens ein unlösliches Konditioniermittel, das unter den Poly-α-Olefinen, fluorierten Ölen, fluorierten Wachsen, fluorierten Gummen und Fettsäureestern ausgewählt ist, enthält, dadurch gekennzeichnet, daß es ferner mindestens einen Ton enthält, wobei das Schaumvermögen der Zusammensetzung über 50 ml liegt.

2. Zusammensetzungen nach Anspruch 1, dadurch gekennzeichnet, daß der Gehalt an Ton im Bereich von 0,5 bis 15 Gew.-%, bezogen auf das Gesamtgewicht der Zusammensetzung, liegt.

3. Zusammensetzungen nach Anspruch 2, dadurch gekennzeichnet, daß der Gehalt im Bereich von 2 bis 10 Gew.-%, bezogen auf das Gesamtgewicht der Zusammensetzung, liegt.

4. Zusammensetzungen nach Anspruch 3, dadurch gekennzeichnet, daß der Gehalt im Bereich von 4 bis 8 Gew.-%, bezogen auf das Gesamtgewicht der Zusammensetzung, liegt.

5. Zusammensetzungen nach einem der vorhergehenden Ansprüche, dadurch gekennzeichnet, daß das oder die grenzflächenaktiven Reinigungsmittel in Anteilen von 5 bis 50 Gew.-% und vorzugsweise von 5 bis 20 Gew.-%, bezogen auf das Gesamtgewicht der Zusammensetzung, vorliegen.

6. Zusammensetzungen nach einem der vorhergehenden Ansprüche, dadurch gekennzeichnet, daß das oder die Konditioniermittel in Anteilen von 0,001 bis 10 Gew.-%, bezogen auf das Gesamtgewicht der Zusammensetzung, vorliegen.

7. Zusammensetzungen nach Anspruch 6, dadurch gekennzeichnet, daß das oder die Konditioniermittel in Anteilen von 0,01 bis 5 Gew.-%, bezogen auf das Gesamtgewicht der Zusammensetzung, vorliegen.

8. Zusammensetzungen nach einem der vorhergehenden Ansprüche, dadurch gekennzeichnet, daß sie einen pH-Wert im Bereich von 4 bis 8 aufweisen.

9. Zusammensetzungen nach einem der vorhergehenden Ansprüche, dadurch gekennzeichnet, daß das oder die grenzflächenaktiven Reinigungsmittel vom anionischen, nichtionischen, amphoteren und zwitterionischen Typ sind.

10. Zusammensetzungen nach einem der vorhergehenden Ansprüche, dadurch gekennzeichnet, daß das oder die grenzflächenaktiven Reinigungsmittel vom anionischen Typ sind.

11. Zusammensetzungen nach einem der vorhergehenden Ansprüche, dadurch gekennzeichnet, daß die grenzflächenaktiven Reinigungsmittel ein Gemisch von anionischen Reinigungsmitteln und amphoteren, zwitterionischen oder nichtionischen Reinigungsmitteln sind.

12. Zusammensetzungen nach einem der vorhergehenden Ansprüche, dadurch gekennzeichnet, daß der Ton aus der Gruppe von Kaolinit, Halloysit, Donbassit, Antigorit, Berthierin, Pyrophyllit, Montmorilloniten, Beidellit, Vermiculiten, Talk, Stevensit, Hectoriten, Saponiten, Chloriten und Sepiolith ausgewählt ist.

13. Zusammensetzungen nach einem der vorhergehenden Ansprüche, dadurch gekennzeichnet, daß der Ton unter Kaolinit, Montmorilloniten, Hectoriten und deren Gemischen ausgewählt ist.

14. Zusammensetzungen nach einem der vorhergehenden Ansprüche, dadurch gekennzeichnet, daß die Poly-α-Olefine vom gegebenenfalls hydrierten Polybutentyp oder vom gegebenenfalls hydrierten Polydecentyp sind.

15. Zusammensetzungen nach einem der vorhergehenden Ansprüche, dadurch gekennzeichnet, daß die Fettsäureester unter Ethylpalmitat, Isopropylpalmitat, 2-Ethylhexylpalmitat, 2-Octyldecylpalmitat, Alkylmyristaten, wie Isopropylmyristat, Butylmyristat, Cetylmyristat und 2-Octyldodecylmyristat, Hexylstearat, Butylstearat, Dioctylmalat, Hexyllaurat, 2-Hexyldecyllaurat und Isononylisononat ausgewählt sind.

16. Zusammensetzungen nach einem der vorhergehenden Ansprüche, dadurch gekennzeichnet, daß die fluorierten Öle, fluorierten Wachse und fluorierten Gummen unter den Perfluorpolyethern und Fluorkohlenwasserstoffverbindungen ausgewählt sind.

17. Verwendung eines Tons als Mittel zur Dispergierung eines Konditioniermittels, das unter den Poly-α-Olefinen, fluorierten Ölen, fluorierten Wachsen, fluorierten Gummen und Fettsäureestern ausgewählt ist, in einer kosmetischen Reinigungszusammensetzung, die mindestens ein grenzflächenaktives Reinigungsmittel enthält.

18. Verwendung einer Zusammensetzung wie nach einem der Ansprüche 1 bis 16 zur Reinigung der Haare, der Kopfhaut und/oder der Haut.
